(19) Europäisches Patentamt
European Patent Office
Office européen des brevets

(11) Publication number: 0 095 833 A2

(12) **EUROPEAN PATENT APPLICATION**

(21) Application number: **83302465.6**

(22) Date of filing: **29.04.83**

(51) Int. Cl.³: **A 61 K 33/00,** A 61 K 31/00, A 61 K 33/02, A 61 K 31/495, C 12 Q 1/70

(30) Priority: **03.05.82 US 374492**

(43) Date of publication of application: **07.12.83 Bulletin 83/49**

(84) Designated Contracting States: **BE CH DE FR GB IT LI SE**

(71) Applicant: **Vilagines, Roland, 34 Avenue de Bellevue, F-91210 Draveil (FR)**
Applicant: **ICS CONSULTING & SERVICE CO. AG., Dammstrasse 29, CH-8702 Zollikon-Zurich (CH)**

(72) Inventor: **Vilagines, Roland, 34 Ave. de Bellevue, F-91210 Draveil (FR)**

(74) Representative: **Dixon, Donald Cossar et al, Gee & Co. Chancery House Chancery Lane, London WC2A 1QU (GB)**

(54) **Method of preventing infection by viropexis-penetrating viruses.**

(57) The class of weakly basic molecules which are at least partially soluble in water, and which are at neutral pH (7 to 7.5) partially or totally under basic form, electrically neutral, gaseous or liposoluble, and which have a pKa greater than the lysosomal pH (5 to 5.5) and which are neither virucidal nor generally cytotoxic provide an important new class of antiviral agents which are capable of inhibiting the penetration of viropexis-penetrating viruses, such as vesicular stomatitis virus (VSV), into the cytoplasmic matrix of target cells which are contacted with the anti-viral agent, preferably before the cell is contacted with the virus. These anti-viral agents do not have any anti-viral effect on fusion-penetrating viruses and, therefore, these anti-viral agents provide a valuable tool for screening viruses by their cell penetrating mechanisms as well as for their own potential therapeutic value.

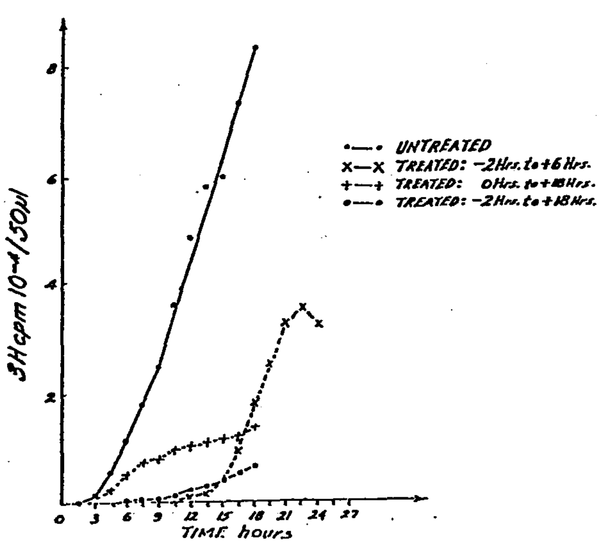


EP 0 095 833 A2

# METHOD OF PREVENTING INFECTION BY VIROPEXIS-PENETRATING VIRUSES

This invention relates to a method of treating human and animal cells which are capable of supporting replication of viropexis-penetrating viruses with a class of chemicals which are neither cytotoxic nor virucidal but which are capable of inhibiting replication of the viropexis-penetrating viruses which come into contact with the cells.

More particularly, this invention relates to the discovery of a class of antiviral agents related by chemical and electrochemical properties rather than by chemical structure analogy which can block the mechanism of replication of viropexis-penetrating viruses by inhibiting the ability of viropexis-penetrating viruses to penetrate into the target cell cytoplasmic matrix.

Viruses are generally categorized into two main classes: viropexis-penetrating viruses and fusion-penetrating viruses, depending on the mechanism by which the virus enters into and infects a target cell. Some viruses appear to be capable of penetrating through the cellular membrane by either of these routes or by a combination of the two mechanisms or by modifications of these mechanisms. For a more thorough review of these penetration mechanisms, the reader is referred to the article by Samuel Dales, "Early Events in Cell-Animal Virus Interactions" Bacteriological Reviews, Vol. 37, No. 2, June 1973, pp. 103-135. The penetration mechanisms of viruses and viral particles are the subject of articles by J. Borsa, et al. "Two Modes of Entry Of Reovirus Particles Into L Cells" J. gen. Virol, Vol. 45,

1979, pp. 161-170 and K. Cooms, et al. "Effects of Chloroquine and Cytochalasin B on the Infection of Cells by Sindbis Virus and Vesicular Stomatis Virus" J. of Virology, Vol. 37, No. 3, March 1981, pp. 1060-1065.

Nevertheless, these penetration mechanisms and the subsequent steps in the viral infection process whereby the viral RNA enters into and modifies the host cell synthesis are still insufficiently understood and have not led to any general methodology for preventing viral infections in higher organisms, including, for example, plants, animals and human.

Thus, for about the last thirty years, research projects for developing antiviral agents which have been based on advances in molecular biology and analogous arts have had only disappointing results. Moreover, attempts to adopt the "structural analogy" principle from study of antibacterial antibiotics have been fraught with failure, largely due to the similarities between viral and cellular synthesis. In view of these similarities substantially all viral development inhibitors are also inhibitors for the corresponding cellular synthesis and, therefore, are useless for therapeutic applications or as clinical research tools.

Accordingly, researchers have long sought after the discovery of agents which are able to selectively block viral development without concomitant inhibition of cellular development. However, it could be by the fortuitous discovery of the antiviral effect of a particular candidate drug for a particular viral agent acting at an unknown level of the viral development sequence, that researchers will be able to obtain new weapons in the arsenal agsint viral infections.

Despite the significant advances in recent years in the fields of cellular biology, microbiology and virology there still have not been many successful attempts to discover a means to block the basic mechanism of viral infection and replication having applicability to a broad class of viruses.

In the above mentioned articles by Coombs, et al, the authors report the following findings:

·When BHK-21 cells (80% confluent) were treated with 0.1 mM chloroquine or 10 μg of cytochalasin B for a period of thirty minutes before infection with Sindbis virus at a multiplicity of 5,000 plaque forming units (PFU) for either 30 or 60 minutes at room temperature in the presence of the drug, cytochalasin B blocked the internalization by endocytosis of the virus (Sindbis virus was not found in coated pits or internal vesicles, although the virions were attached to the cell surface) while internalization was either enhanced or took place normally in the presence of chloroquine;

·When monolayers of BHK-21 cells were treated with chloroquine (0.1M) or cytochalasin B (10 μg/ml) and the treated layers were then infected with vesicular stomatitis virus (VSV or Sindbis virus at 50 PFU per cell) for 1 hour and incubated for 12 hours the yields of VSV and Sindbis virus were reduced in the chloroquine treated cells but the yields were the same as untreated (control) cells in the case of cytochalasin B.

From these observations, the authors concluded that they were able to confirm earlier reports, see e.g. the review by Samuel Dales referred to above, that chloroquine treatment of cultrued cells can inhibit replication of VSV or Sindbis virus, although endocytosis of Sindbis virus by cells can take place in the presence of chloroquine. The inhibited replication of Sindbis virus in cells that become infected in the presence of chloroquine is suggested to be due to "blocking some late step in maturation." It is also hypothesized that in the high infection rate experiments (i.e. 5,000 PFU per cell) the route of successful infection is not dependent upon ingestion of the particles by the cells and is taken by only a non-detectable minority of virions. It is further suggested that this successful route of infection may be by direct fusion of the virion membrane with the surface of the host cell and

chloroquine may block this fusion by altering characteristics of the cell plasma membrane. Finally, the authors state that their "data are incompatible with the concept that internalization of virus by phagocytosis, followed by an interaction of lysosomal structures with the resulting phagosomes, is essential for the infection of cells."

According to the best present knowledge of microbiologists and virologists, the penetration of viropexis-penetrating viruses into cellular cytoplasmic matrix proceeds by the following steps: the viropexis-penetrating viruses which adhere to the cell surface (membrane) are engulfed in a phagocytic vacuole and internalized therein; the phagocytic vacuole then associates itself with a "primary" lysosome in order to induce a new intra-cellular organelle - the "secondary" lysosome; enzymes of the primary lysosome are then discharged onto the internalized virus which is, at least partially, uncoated; after lysis of the membrane of the secondary lysosome viral cores and uncoated viral nucleic acid are liberated into the cytoplasmic matrix of the host cell.

It has now been discovered that the ability of viropexis-penetrating viruses to infect a cell can be inhibited by treating the cell with a class of substances which, although non-virucidal and preferably non-cytotoxic can block this mechanism in a reversible manner at the level of the lysosome. More particularly, the members of this class of at least slightly water soluble substances are characterized by being slightly basic, i.e. exhibiting electrical neutrality at a pH in the range of from about 7 to about 7.5, preferably gaseous or liposoluble at said pH, and positively charged at and having a pKa larger than, the pH of the lysosomes, i.e. pH about 5 to 5.5, or by being capable of generating

*in situ* compounds satisfying these criteria.

Based on this discovery, the present invention has provided a method of preventing a viropexis-penetrating virus from entering into the cytoplasmic matrix of a cell capable of supporting replication of the viropexis-penetrating virus by contacting the target cell with a member of the above described class of antiviral agents, preferably prior to the cell coming into contact with the viropexis-penetrating virus.

Although not wishing to be bound to any specific theory of the anti-viral behavior of these substances, it is hypothesized, consistent with the data to be given below, that the anti-viral agents block the fusion between the primary lysosome and the phagocytic vacuole. This blockage is due to the diffusion of the basic, electrically neutral form of the molecule into the lysosome where, because of the lower pH of said lysosome, it captures a proton, becomes positively charged and then it interacts with the negatively charged intra-lysosomal proteins. This interaction creates a stabilized lysosome which is thereby prevented from associating itself with the phagocytic vacuole containg the virus.

In view of the selectivity of this class of anti-viral agents in inhibiting replication of viropexis-penetrating viruses but not of fusion-penetrating virus, the present invention provides an analytical tool for screening previously unclassified viruses or viruses whose classifications are questionable, to determine if it is viropexis-penetrating (inhibiting penetration) or partially viropexis-penetrating (partially inhibiting penetration), or fusion-penetrating (not inhibiting penetration), for any particular cell line. In addition, the anti-viral agents of this invention have strong potential therapeutic effect for preventing or treating viral infections.

In the accompanying drawings:

FIGURE 1 is a plot of RNA synthesis of ammonium chloride treated and untreated cells as a measure of cytotoxicity taken from Example IV;

FIGURE 2 is a plot of RNA released by cells infected by VSV taken from Example V;

FIGURE 3 is a plot of the zonal gradient analysis from Example VI;

FIGURE 4 is a plot of the zonal gradient analysis from Example VI using a different ordinate scale;

FIGURE 5 is a plot of the zonal gradient analysis, seven hours after inoculation, of cytoplasmic extracts of cells infected with highly radioactive VSV, treated with 20 mM $NH_4Cl$ from the second hour before inoculation or untreated taken from Example VII;

FIGURE 6 is a plot of the zonal SDS - Sucrose gradient analysis, 18 hours after infection of cytoplasmic extracts of VSV infected cells untreated (panel A) or treated with 20 mM $NH_4Cl$ from the second hour before inoculation to the sixth hour after (panel B), taken from Example VII;

FIGURE 7 is a zonal SDS - Sucrose gradient analysis, 18 hours after infection of cytoplasmic extracts of VSV infected cells treated by 20 mM $NH_4Cl$ from the second hour before inoculation to the eighteenth hour after (panel A) or from immediately after inoculation to the eighteenth hour after (panel B) also taken from Example VII; and

FIGURE 8 is a plot of the equilibrium curves of $NH_4OH$, $NH_3$ and $NH_4^+$.

Embodiments of the present invention will now be described, with reference to the said accompanying drawings.

Viropexis-penetrating viruses depend on their interaction with a phagocytic vacuole to penetrate through the cell membrane into the cytoplasmic matrix. In contrast, fusion-penetrating viruses appear to directly "melt"

their way through the cell membrane. While most viruses have been found to penetrate into the cytoplasmic matrix through one or the other of these mechanisms some viruses have been shown to penetrate by both of these mechanisms. The present invention can be applied to any virus which depends, at least in part, to the viropexis-penetration route to pass through the cell plasma membrane into the cytoplasmic matrix, although the best and most efficient results are, of course, achieved when applied to viruses which penetrate solely by viropexis.

Among the viropexis-penetrating viruses for one or more different cell types mention can be made, for example, of herpes agents such as Herpes simplex and equine abortion virus, pox viruses, such as Molluscum contagiosum and melolontha; adenoviruses, such as $SV_{15}$ and Human 1, 5, 7 and 12 viruses; papovaviruses, such as polyoma and $SV_{40}$; reoviruses, such as Type 3; Rhabdoviruses, such as VSV and rabies, myxoviruses, such as inflenza, influenza A, and influenza WSN; and paramyxoviruses, such as Sendai, $SV_5$, and Avian sarcoma and leukosis viruses.

Morever, virtually any cell type that is capable of supporting replication of the virus can be treated by the anti-viral agents of this invention to prevent the viropexis penetrating virus from entering into the cytoplasmic matrix. Thus, human cells, animal cells, plant cells and insect cells can all be treated and similarly, among these sources any cell capable of being infected can also be treated.

Since only relatively few of the known viruses have been sufficiently studied to ascertain their penetration routes into the many different types of cell lines and since it is known that the anti-viral agents of this invention are specific for viropexis-penetrating viruses, the methodology of this invention among other

applications is useful as an analytical tool for screening viruses to determine whether or not they are at least partially or totally viropexis-penetrating viruses. Thus, if a particular virus is known to be able to replicate in a specific cell type, and if replication, as determined, for example, by measuring viral nucleic acid synthesis, is prevented when the cells are preferably first contacted with an anti-viral agent of this invention, then viropexis is the penetrating mechanism. If, on the other hand, replication is not prevented, or if replication is partially inhibited, then penetration is not by viropexis or is only partially by viropexis and partially by an alternative route, e.g. fusion.

The anti-viral agents of this invention are at least slightly soluble in water, slightly basic and non-ionized at neutrality (pH from 7 to 7.5) and have a pKa greater than, but preferably close to, the pH of the lysosomes of cells (about 5 to 5.5).

The anti-viral agents must also have a good diffusion or be at least partially soluble in the cytoplasmic matrix of the target cells and for this purpose liposoluble substances or gaseous molecules are satisfactory. Molecules of relatively small molecular size are preferred since such molecules can most readily pass through the cell plasma membrane into the cytoplasmic matrix. In addition, the anti-viral agents must be non-toxic to the target cell as well as to the host organism, e.g. plant, animal, human, etc., if the anti-viral agent is to be used in <u>in vivo</u> applications.

As stated above, the anti-viral agents of this invention are not virucidal, i.e. they do not directly destroy the virus, but rather interfere with the viral penetration process. However, to the extent that any particular compound having the other properties of the anti-viral agents of this invention may also have a virucidal and/or a slight cytotoxic effect such compound can also be used in the method of this invention.

The anti-viral compounds include both inorganic compounds and organic compounds. Among the inorganic compounds mention can be made of, for example, inorganic bases such as ammonium hydroxide and calcium hydroxide; inorganic salts, such as ammonium chloride. Organic compounds having pKa's in excess of pH 5 to 5.5 include, for example, organic bases, such as aminoethyl alcohol, apomorphine, betaine, brucine, choline, cocaine, codeine, hydroquinine, DL hystidine, lycine, morphine, nicotine, novocain, papaverine, phenylguanidine, physostigmine, piperazine, quinidine, quinine, tebaine and veronal; and organic indicators such as bromothymol blue, phenol red, thymol blue, neutral red, cresol red, α-naphthophthalein, phenolphthalein, thymol phthalein and dithizone

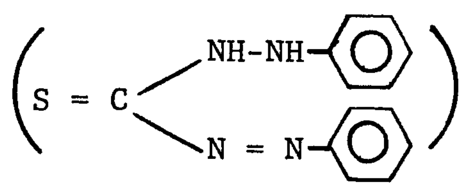

The amount of the anti-viral agent to be brought into contact with the target cells need be only that amount sufficient to provide neutralization of all the negatively charged lysosomal molecules of lysosome organelles in the target cells. That is, in view of the hypothesized mechanism of action of the anti-viral agent as reacting with the negatively charged lysomal proteins to form a stabilized complex without any "free" lysosomes the amount of the anti-viral agent should be sufficient to neutralize *in situ* the target-lysomal proteins.

In *in vitro* culture systems the anti-viral agent can be added to the culture medium any time prior to inoculation with the viropexis-penetrating virus, preferably from about 10 hours to about 10 minutes, more preferably from about 2 hours to about 30 minutes, before inoculation.

In *in vivo* applications, the anti-viral agent can be administered by any convenient route, for example, intranasal spray, oral liquid, tablet or powder, intraveinous and intra-peritoneal. Preferably, the agent will be administered prior to anticipated infection with the virus, although growth of infection can be reduced by administering a dose of the anti-viral agent after the initial viral infection.

EXAMPLE 1

This example shows the inhibition of the development of different viruses by ammonium chloride. Cells were treated for 2 hours before inoculation with 20 mM $NH_4Cl$ in a liquid culture medium. After inoculation, in the presence of $NH_4Cl$, the cells were overlaid by agar culture medium adjusted to 20 mM $NH_4Cl$. The cytopathic effect was determined, and PFU number at the end of the one step growth for each virus was measured.

The result are shown in Table I.

TABLE I

Action of 20 mM $NH_4Cl$ on the development of different viruses

| Cells | Viruses | Virions produced in untreated infected cells | Virions produced in infected 20mM $NH_4Cl$* treated cells |
|---|---|---|---|
| HeLa | VSV | $1.3 \times 10^8$PFU/ml | OPFU/ml |
| L | Vaccinia virus | $2.9 \times 10^6$PFU/ml | $3.5 \times 10^6$PFU/ml |
| L | Reovirus III | $3.16 \times 10^8$PFU/ml | OPFU/ml |
| BGM | Polio virus | $3.6 \times 10^8$PFU/ml | $4.15 \times 10^8$PFU/ml |

*Lowest dose giving maximum anti-viral effect

Thus, 20 mM $NH_4Cl$, added from 2 hours before inoculation completely inhibits the development of VSV and Reovirus. No action on the viral development of Vaccinia or Polio virus is observed.

Identical results are obtained in L, BHK, and BGM cells for VSV; and in BGM cells for Reovirus III.

EXAMPLE II

This example shows that this effect is reversible. Two batches of cells were treated by an identical concentration (20mM) of $NH_4Cl$ applied respectively either 2 hours before VSV inoculation or immediately after. A third batch was only treated from the second hour before to the sixth hour after inoculation. The fourth, control, batch was treated by 20 mM NaCl. At various times the virus was titrated in the supernatants. The results are summarized in Table II.

TABLE II

Reversibility of $NH_4Cl$ effect
VSV development

| Treatm. Condtns. \ Time of Titr. (PFU/ ml) | 0 h | 6 h | 18 h | 24 h |
|---|---|---|---|---|
| None (control) | $2.88 \times 10^3$ | - | $3.32 \times 10^8$ | |
| 0 h - 18 h | $7.40 \times 10^3$ | - | $3.45 \times 10^8$ | |
| -2 h - 18 h | $5.20 \times 10^3$ | - | $5.03 \times 10^5$ | |
| -2 h - 6 h | $4.9 \times 10^3$ | $3.32 \times 10^3$ | | $9.8 \times 10^8$ |

The differences observed between the times 0 h and 18 h in the infected cells treated from the second hour before to the eighteenth hour after can be attributed, as will be discussed later, to the release of parental virions stored into the treated cells rather than to a real viral multiplication.

EXAMPLE III

This example shows the lack of virucidal effect of $NH_4Cl$. A suspension of VSV was divided into 3 batches. The first batch was diluted with Phosphate Buffered Saline (PBS) and adjusted to a final concentration of 20 mM with $NH_4Cl$. The second batch (control 1) received the same volume of PBS and was adjusted to 20 mM with NaCl. The third batch (control 2) received the same volume of PBS. The three batches were then incubated at 37°C. Aliquots of each batch were titrated at various time. The results are summarized in Table III.

TABLE III

Study at 37°C of the virucidal effect of $NH_4Cl$

| Medium / Time of incub. at 37°C | PBS | PBS + NaCl | PBS + $NH_4Cl$ |
|---|---|---|---|
| 0 h | 3.45 x $10^8$PFU/ml | 4.2 x $10^8$PFU/ml | 3.5 x $10^8$PFU/ml |
| 2 h | 4.62 x $10^8$PFU/ml | 4.08 x $10^8$PFU/ml | 4.07 x $10^8$PFU/ml |
| 6 h | 2.75 x $10^8$PFU/ml | 3.95 x $10^8$PFU/ml | 4.8 x $10^8$PFU/ml |
| 24 h | 8.8 x $10^8$PFU/ml | 8.95 x $10^8$PFU/ml | 7.8 x $10^8$PFU/ml |

It can be seen that $NH_4Cl$ has no virucidal effect against VSV.

EXAMPLE IV

This example shows that $NH_4Cl$ has no cytotoxic effect. The principle of the well-known, very sensitive, method used consists in the comparison of the RNA synthesis behavior in cells treated for 24 hours by a drug to the RNA synthesis behavior in untreated cells. The results are summarized in Figure 1.

Figure 1 shows that after a 24 hour contact time $NH_4Cl$ has no cytotoxic effect. This result is in agreement with the normal development of Vaccinia and Polio virus in treated cells (TABLE I) showing, once again, that cellular synthesis can proceed in the presence of the anti-viral agent.

The preceding examples show that:

- a 20 mM concentration of $NH_4Cl$ completely blocks the Reovirus and VSV development
- $NH_4Cl$ has no action on the Polio virus and Vaccinia virus developments
- $NH_4Cl$ has no cytotoxic effect.

The striking antiviral action demonstrated by $NH_4Cl$ suggests that the anti-viral agent interacts with a very specific biochemical step of VSV and Reovirus development which does not seem to exist in Vaccinia and Polio virus development. On the basis of current knowledge the only step which could seriously account for such a difference is the penetration step. Although there might be some conflicting evidence, it is presumed that Vaccinia and Polio virus penetrate by fusion with the plasma membrane whereas VSV and Reovirus penetrate by viropexis. $NH_4Cl$ seems therefore to inhibit selectively the viral development of viropexis-penetrating viruses.

In the following example, this hypothesis is shown to be consistent with the obtained data.

EXAMPLE V

This example shows the kinetics of the release of viral RNA in the supernatant of infected cells treated or untreated by ammonium chloride. Two batches of HeLa cells were treated with 20 mM $NH_4Cl$ from respectively 2 hours before and immediately after VSV inoculation until 18 hours after inoculation. A third batch was treated from 2 hours before inoculation to the sixth hour after. The fourth, control, batch received 20 mM NaCl. The infected cells were labelled with $^3$H-Uridine immediately after inoculation and radioactivity was measured at various times in aliquots of supernatants. The results were plotted in Figure 2.

From Figure 2, the following observations can be made:

- Viral RNA synthesis, which increases drastically in infected untreated cells, is seriously reduced in cells treated with $NH_4Cl$ from 2 hours before to 18 hours after inoculation.

- Withdrawal of $NH_4Cl$ at the sixth hour after inoculation induces a 6 hour lag period followed by a rather normal viral RNA synthesis.

- When $NH_4Cl$ is only added after inoculation, the viral RNA synthesis, even though strongly reduced, is more important than it is when this product is maintained from the second hour before inoculation to the eighteenth hour after.

Taking into account the fact that the appearance of RNA in the supernatant of infected cells treated by $NH_4Cl$ from the second hour before to the eighteenth hour after inoculation, can be exclusively attributed to a release of cellular RNA, these results are in agreement with the preceding data showing that the anti-viral agent has a reversible inhibitory effect on VSV development.

 

EXAMPLE VI

This example studies the appearance of virions in the culture medium of infected cells treated or untreated by $NH_4Cl$.

The culture medium of $NH_4Cl$ treated or untreated cells under the conditions previously described in Example V were analyzed by zonal sucrose gradient analysis at the end of 18 hours after inoculation. The results were plotted in Figure 3.

From panel A, it is seen that the supernatant of untreated cells contains a large amount of viral cores (120 S) and virions (625 S). The supernatant of $NH_4Cl$ treated cells from the second hour before to the sixth hour after inoculation, contains a lower amount of virions, even though not negligible.

From panel B, it is seen that the supernatant of cells treated by $NH_4Cl$ immediately after inoculation, also contains viral cores even though at a lower level than in the supernatant of untreated cells. It can also be seen that the supernatant of cells treated by $NH_4Cl$ from the second hour before inoculation to the eighteenth hour after contains neither viral cores nor virions, the slight labelling observed in the top of the gradient being ribosomal RNA.

In order to further clarify the reversible inhibitory effect of $NH_4Cl$ on virion production the data from the first 15 fractions of the zonal gradient analysis for each of the four samples is plotted in Figure 4 using an expanded ordinate scale.

EXAMPLE VII

In this example, the fate of VSV in the cytoplasmic extracts of infected cells treated or untreated by $NH_4Cl$ was followed and the different classes of viral messengers - RNA's synthesized under the test conditions were also measured.

Cytoplasmic extracts of cells treated or untreated by $NH_4Cl$ from the second hour before infection by highly radioactive VSV to the seventh hour after, were analyzed, at the end of the seventh hour by zonal SDS-sucrose gradient analysis: 15-30% w/v sucrose, 30 mn, 35 Krpm. The results are plotted in Figure 5.

It can be seen that the cytoplasmic extract of untreated cells (panel B) contains a large amount of viral cores (120s). On the contrary, cytoplasmic extract of cells treated from the second hour before inoculation to the seventh hour after (panel A) contains a negligible amount of viral cores and a large amount of complete virus (625S).

These results show that the parental viral particles from the inoculum are still intact, in the cytoplasmic extract of infected treated cells. The conclusion of this is that the anti-viral agent inhibits the penetration of VSV into the cytoplasmic matrix of cells and, therefore, its uncoating, i.e. the virus remains intact inside the phagocytic vacuole and does not multiply.

The different classes of cellular and viral m-RNAs present at the eighteenth hour after infection in the cytoplasmic extracts of cells treated or untreated as described above were analysed by sucrose-SDS gradients. The results are plotted in Figure 6 for the untreated cells and cells treated from the 2nd hour before to the 6th hour after inoculation and in Figure 7, for cells treated either from 2 hours before, or immediately after (time 0) inoculation to 18 hours after inoculation.

From Figure 6, panel A, it is seen that infected untreated cells contain, 18 hours after infection, a large amount of 42S viral RNA. The presence of large amounts of L viral protein 31S m RNAs, G viral protein 17S m RNAs, N viral protein 14.5S m RNAs, and M and NS viral proteins 12S m RNAs can also be noticed as well as the presence of 4S transfer RNA.

Panel B, Figure 6 shows the different classes of RNA present 18 hours after infection in the cytoplasmic

extracts of infected cells treated with 20 mM $NH_4Cl$ from the second hour before infection to the sixth hour after. The onset of viral infection being delayed for 6 hours, the amount of 42S mature viral RNA is normally lower than in untreated cells and, on the contrary, there are more viral m RNAs responsible for the synthesis of the different viral proteins: 31S, 17S, 14.5S, and 12S m RNAs.

Figure 7 shows the different classes of RNAs present 18 hours after infection, in the cytoplasmic extracts of infected cells treated by 20 mM $NH_4Cl$ either from the second hour before infection to the eighteenth hour after (panel A) or immediately after infection to the eighteenth hour after (panel B).

These results show (panel A) that the cytoplasmic extracts of $NH_4Cl$ treated cells from the second hour before infection to the eighteenth hour after:

·do not contain 42S viral RNA,

·do not contain viral m RNAs.

On the contrary, the presence of all cellular RNAs: 28S and 18S ribosomal RNAs, 9S m RNAs and 4S transfer RNAs can be noticed.

This result clearly demonstrates that in the presence of $NH_4Cl$ the infection does not proceed because it would rapdily have blocked cellular RNA synthesis. This is consistent with the results shown in connection with Figure 5 and demonstrates once again that VSV was not able to reach the cytoplasmic matrix. This result also confirms the results obtained in Example IV and Figure 1 where it is shown that $NH_4Cl$ has no effect on cellular RNAs synthesis.

In the case of infected cells treated from immediately after inoculation to the eighteenth hour after (panel B) there is a slight reduction of the infection characterized by a large amount of viral m RNAs and a lower viral 42S mature RNA at a time after infection where, in infected untreated cells, the ratio between

these 2 RNAs species is normally inverted. This result is in agreement with those obtained and described in Example V and figure 2. It can also be observed that the few viral RNA synthesized are almost as infectious as the large amount produced in untreated cells (see Table II).

These examples demonstrate that the anti-viral agent completely inhibits the VSV development, its action is reversible and it has no cytotoxic effect.

The results also show that the block of the viral infection is due to the inhibition of the penetration of viral particles into the cytoplasmic matrix of the host cell because, in treated cells:

- viral particles are not lyzed;
- there is no synthesis of any one of the viral m RNAs'
- there is no early block of cellular synthesis as it normally occurs in infected untreated cells.

All of these results are consistent with the conclusion that $NH_4Cl$ selectively inhibits the specific behavior of viropexis-penetrating viruses. As only intact particles can be found in infected treated cells and as no cores seem to be formed de-novo the inventor's presumed mode of action of the anti-viral agent, namely, blocking the fusion between the primary lysosome and the phagocytic vacuole, is consistent with the observed data. This block, in the case of $NH_4Cl$ must be due to the diffusion of $NH_3$ into the lysosomes. This gas, at the lysosomal pH (5 to 5.5) is positively ionized into $NH_4^+$ which interacts with the negatively charged intra-lyosomal proteins leading to a stabilized lysosome which then cannot associate itself with the phagocytic vacuole containing the virus.

Considering further these observations, it is seen that at pH about 7.5, corresponding to the pH of the culture medium, only about 2% of $NH_3$ is formed (see

Fig. 8) and this could explain the rather important inhibitory dose of $NH_4Cl$ (20 mM).

However, the fact that the active component of the molecule in a gas explains, despite the small amount of electrically neutral molecules formed, its excellent diffusion through the different cellular and lysosomal membranes.

More generally, every slightly basic molecule at least partially soluble in water, preferably non-toxic, which is gaseous or soluble into lipids, non-ionized at neutrality ($7<pH<7.5$) and becoming ionized (+) at the lysosomal pH will be able to neutralize the negatively charged lysosomal proteins and, therefore, inhibit its association with the phagocytic vacuoles. The most efficient of the anti-viral compounds with a pKa greater than the pH of the lysosome are those which are most susceptible to penetrate into the cytoplasmic matrix and which pKa will be closer to the lysosomal pH, preferably a pKa of about 5.6 to about 12, preferably pKa of about 5.6 to about 9.

EXAMPLE VIII

This example shows the anti-viral activity of an organic compound meeting the preceding criteria: Neutral Red.

Two batches of cells were pretreated for 2 hours by Neutral Red at final concentrations of, respectively 70 µM and 100 µM. After inoculation by VSV in the presence of Neutral Red, the cells monolayers were layered with agar-medium containing this product at the indicated dose. A third, control, batch was treated under the same conditions by NaCl. Plaques were numbered at the end of the one-step growth (18 hours). The results are summarized in Table IV.

TABLE IV

Action of Neutral Red on the growth of VSV

| Concentrations of Neutral Red | Titer obtained (PFU/ml) |
|---|---|
| 0 µM | $1.5 \times 10^8$ |
| 70 µM | $9.0 \times 10^7$ |
| 100 µM | 0 |

100 µM Neutral Red (29 µg/ml) completely inhibits the growth of VSV. By following the procedure of Example III, it is shown that this product had no virucidal effect at these concentrations. The highly significant difference observed between two concentrations as close as 70 µM and 100 µM can be explained by the proposed level of action which requires a total block of absolutely all the lysosomes. As long as this complete block is not obtained viral infection leads, as it does, in the case of $NH_4Cl$, to a lower production of more infectious viral particles (see Example VII). These results again confirm the action of the anti-viral agents at the level of the lysosome because their normal burst in the cytoplasm of infected untreated cells liberates their RNAse which degrades a not negligible amount of the viral neo-synthesized RNA, limiting in this way the virions production. In the case of insufficient treatment, or a treatment applied only after inoculation, the partial stabilization of lysosomes decreases the amount of liberated lysosomal RNAse allowing the small amount of viral

RNA synthesized to be coated in its entirety leading to the production of an amount of progeny virions almost as important as in untreated cells.

CLAIMS

1. A method of preventing a viropexis-penetrating virus from entering into the cytoplasmic matrix of a cell capable of supporting replication of the viropexis-penetrating virus by contacting the target cell with a non-virucidal, at least partially water soluble anti-viral agent, said agent being partially or totally under basic form electrically neutral and gaseous or liposoluble, at a pH in the range of from about 7 to about 7.5, positively charged to a pH in the range of from about 5 to about 5.5, and having a pKa greater than the pH of the lysosomes of said target cells, whereby said agent will penetrate through the cell membrane of said target cell and come into come into contact with and penetrate into the cell lysosomes to thereby prevent said virus from reacting with said lysosomes.

2. The method of claim 1 wherein the anti-viral agent is used in an amount sufficient to react with at least substantially all of the efficient negatively-charged lysosomal proteins of the target cells.

3. The method of claim 1 wherein the viropexis-penetrating virus is either Vesicular Stomatitis Virus or Reovirus and the anti-viral agent is ammonium chloride, preferably used in an amount of at least about 20mM.

4. The method of claim 1 wherein the anti-viral agent is Neutral Red, preferably used at a concentration of at least about 100µM.

5. The method of claim 1 wherein the anti-viral agent is non-cytotoxic or slightly cytotoxic.

6. The method of claim 1 wherein the target cell is brought into contact with the anti-viral agent before the cell comes into contact with the viropexis-penetrating virus.

7. The method of claim 1 wherein the target cell is brought into contact with the anti-viral agent after the cell comes into contact with the viropexis-penetrating virus whereby the growth of viral infection can be reduced.

8. An agent for use in preventing a viropexis-penetrating virus from entering into the cytoplasmic matrix of a cell capable of supporting replication of the viropexis-penetrating virus,by contacting the target cell with said agent, characterised in that said agent comprises a non-virucidal, at least partially water soluble anti-viral agent, which is partially or totally under basic form electrically neutral and gaseous or liposoluble, at a pH in the range of from about 7 to about 7.5, positively charged to a pH in the range of from about 5 to about 5.5, and having a pKa greater than the pH of the lysosomes of said target cells, whereby said agent will penetrate through the cell membrane of said target cell and come into contact with and penetrate into the cell lysosomes to thereby prevent said virus from reacting with said lysosomes.

9. The anti-viral agent for use as claimed in Claim 8, and comprising ammonium chloride preferably used in an amount of at least about 20mM, or Neutral Red preferably used at a concentration of at least about 100µM.

10. The anti-viral agent for use as claimed in Claim 8, and being non-cytotoxic or slightly cytotoxic.

11. The anti-viral agent for use as claimed in Claim 8, and adapted for convenient administration substantially as hereinbefore described, for example by intra-nasal spray, oral liquid, tablet or powder.

12. The features herein described, or their equivalents, in any patentably novel selection.

0095833

Fig. 1

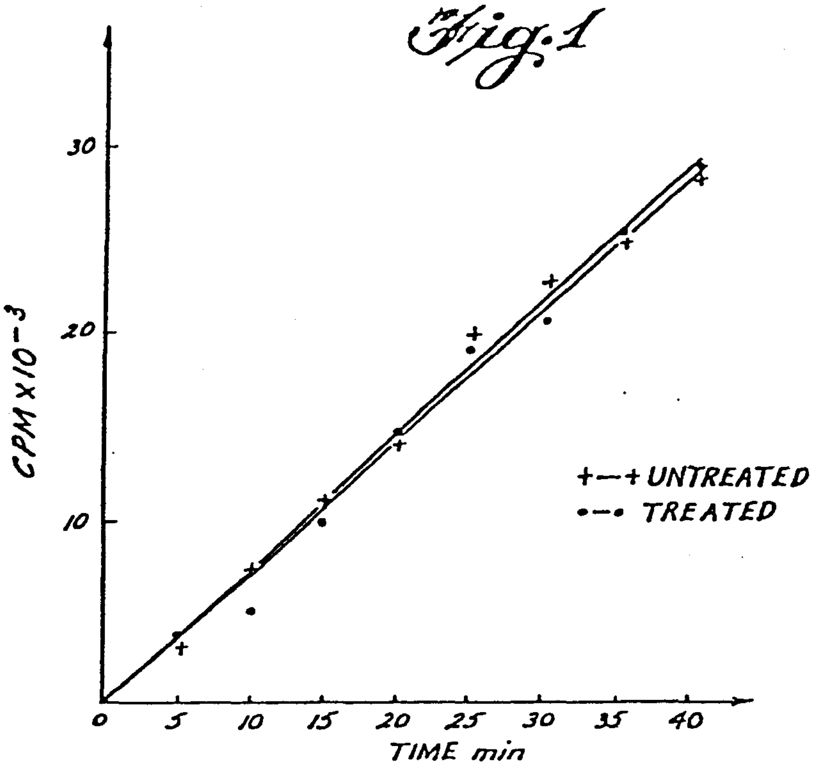


Fig. 2

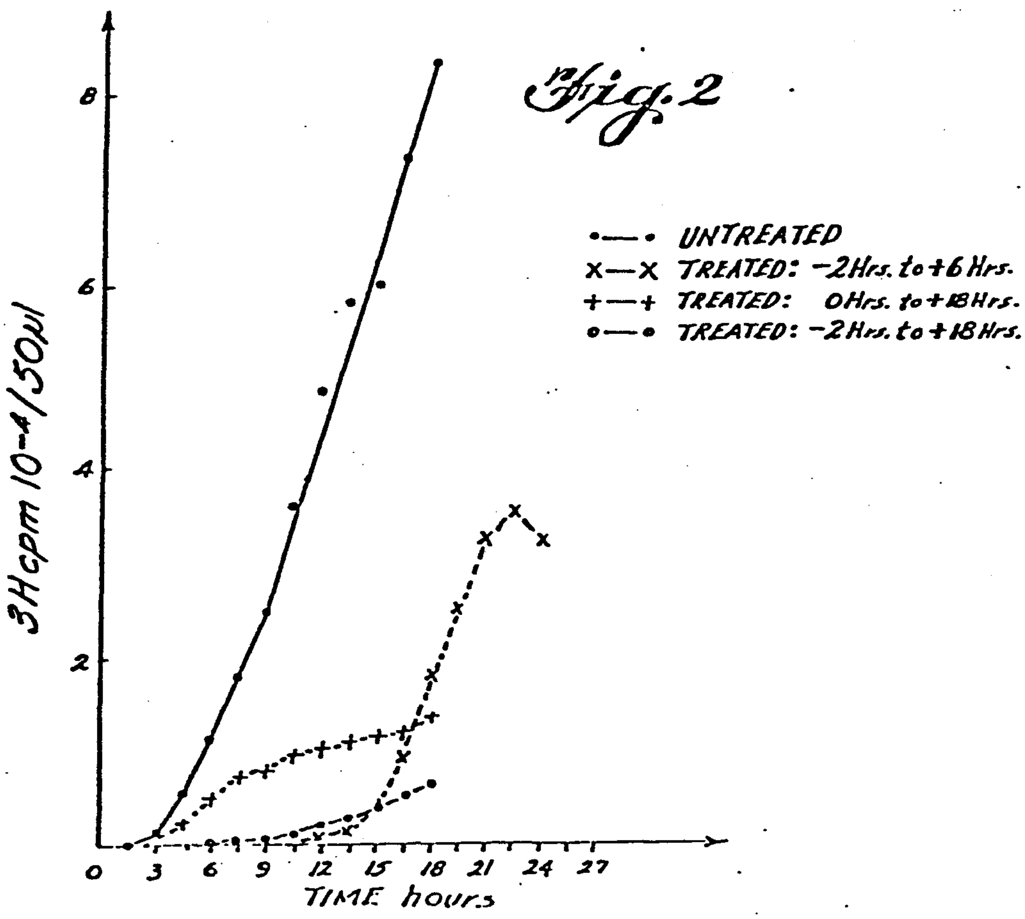

Fig. 4

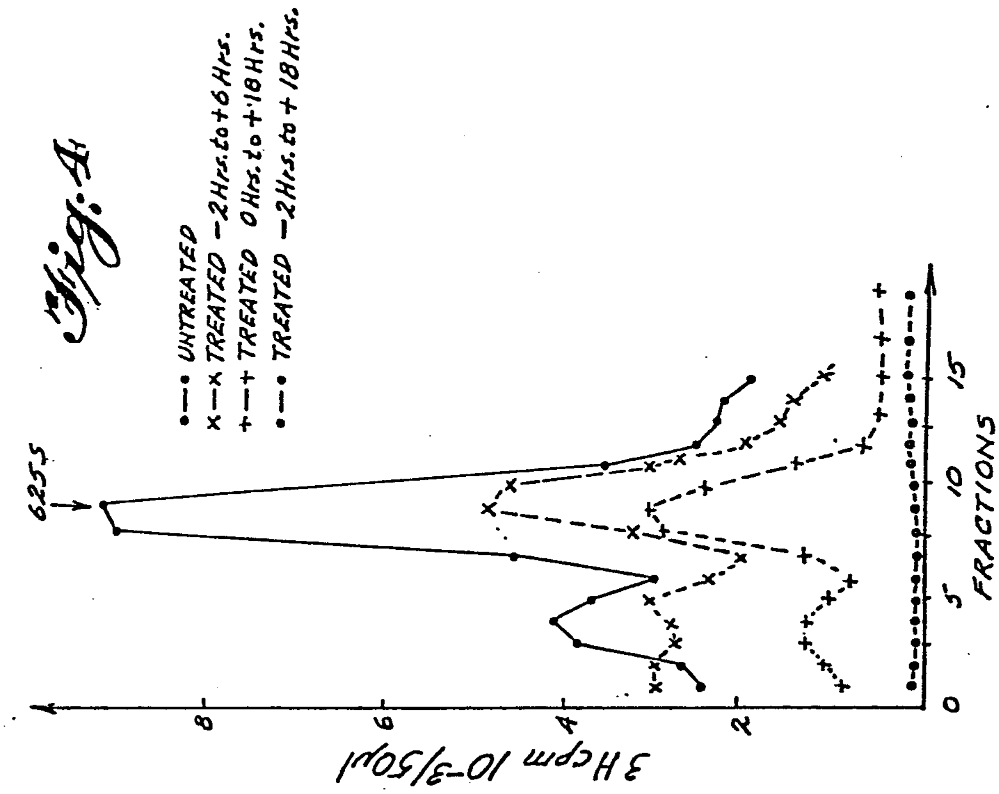

UNTREATED
TREATED −2Hrs.to+6Hrs.
TREATED 0Hrs.to+18Hrs.
TREATED −2Hrs.to+18Hrs.
62.5S
3H cpm 10-3/50µl
FRACTIONS


Fig. 3

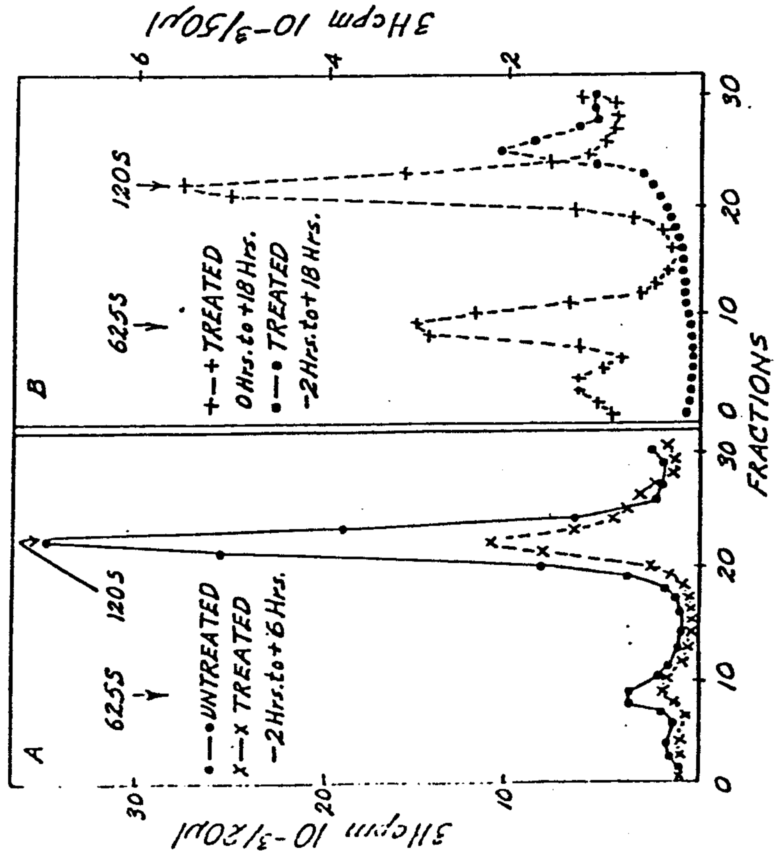

A
B
62.5S
120S
UNTREATED
TREATED −2Hrs.to+6Hrs.
TREATED 0Hrs.to+18Hrs.
TREATED −2Hrs.to+18Hrs.
3H cpm 10-3/20µl
3H cpm 10-3/50µl
FRACTIONS

Fig. 5

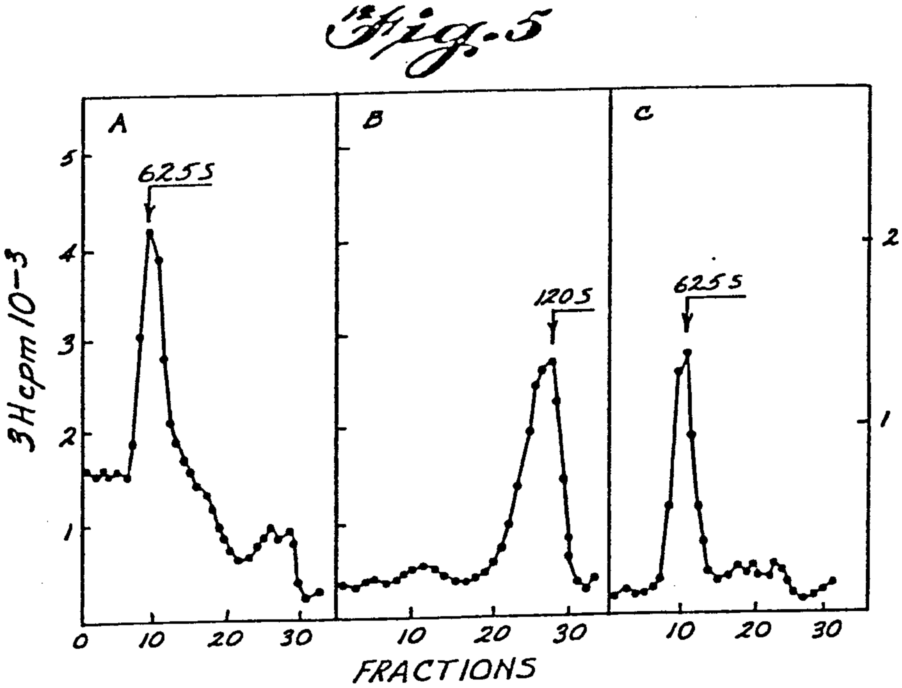
A
B
C
625 S
120 S
625 S
3H cpm 10-3
FRACTIONS

Fig. 6

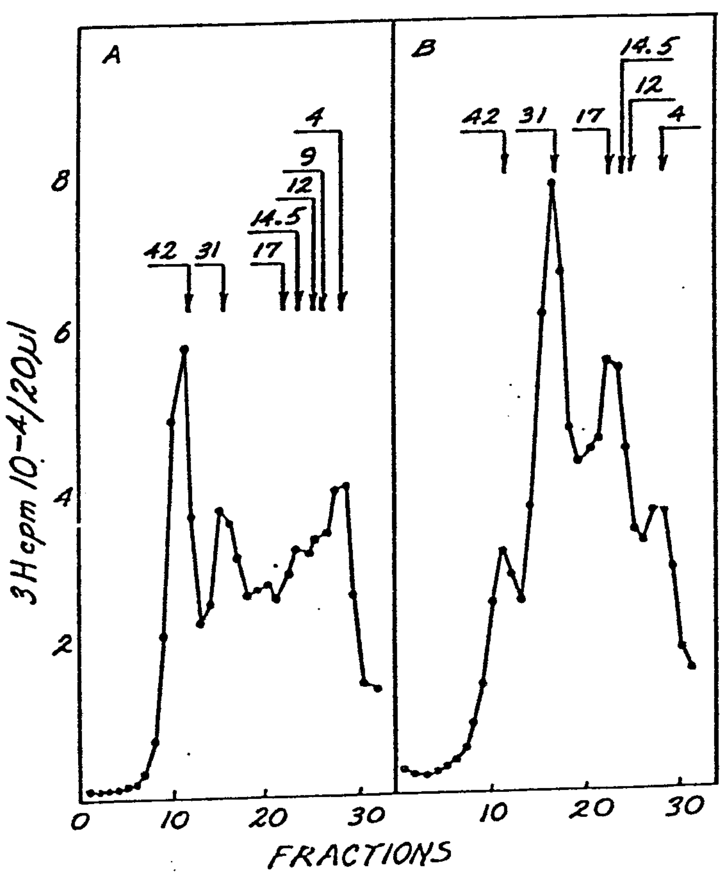
A
B
42
31
17
14.5
12
9
4
3H cpm 10-4/20μl
FRACTIONS

Fig. 7

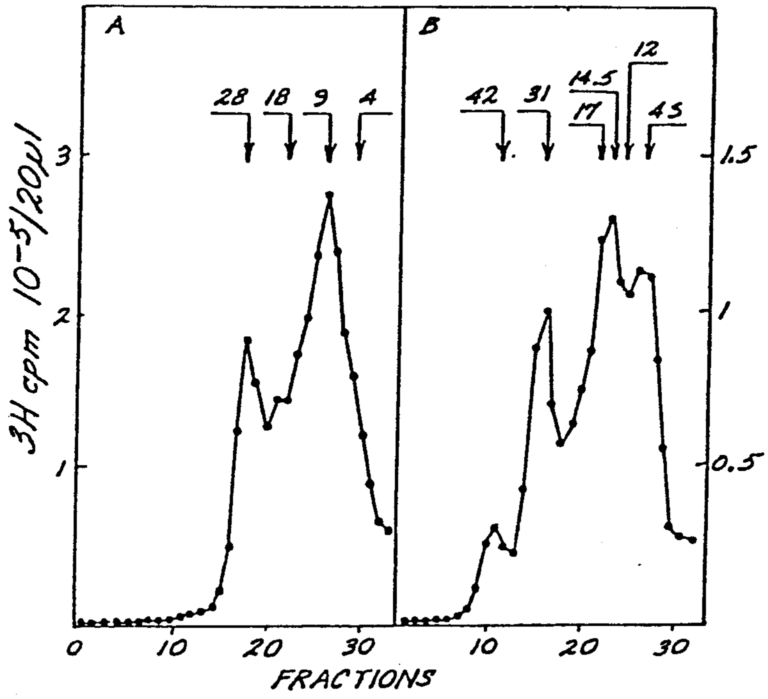
A
B
28
18
9
4
42
31
14.5
17
12
4S
3H cpm 10-5/20µl
3
2
1
1.5
1
0.5
0
10
20
30
FRACTIONS

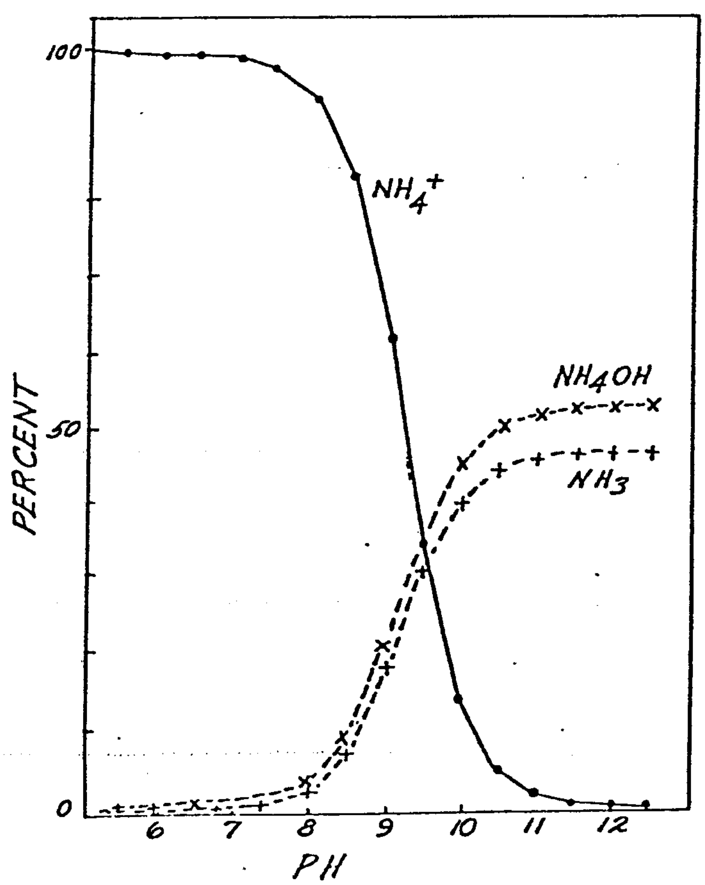
100
50
0
PERCENT
NH4+
NH4OH
NH3
6
7
8
9
10
11
12
PH

Fig. 8